# EUROPEAN PATENT SPECIFICATION

(11) **EP 1 648 515 B1**
(45) Date of publication and mention of the grant of the patent: **21.11.2012**
(21) Application number: 04763163.5
(22) Date of filing: 10.07.2004
(51) Int. Cl.: A61K 45/06, A61K 31/404, A61P 9/12, A61K 31/4184, A61K 9/20

(54) **CHLORTHALIDONE COMBINATIONS**
CHLORTHALIDON-KOMBINATIONEN
COMBINAISONS DE CHLORTHALIDONE

(30) Priority: 16.07.2003 US 487835 P
(43) Date of publication of application: 26.04.2006
(73) Proprietor: Boehringer Ingelheim International GmbH, 55216 Ingelheim am Rhein (DE); Boehringer Ingelheim Pharma GmbH & Co. KG, 55216 Ingelheim (DE)
(72) Inventor: BRAND, Gerrit, Toronto, Ontario M5A 4T4 (CA); BAKRIS, George, L., Munster, IN 46321 (US); DAVIDAI, Giora, New Canaan, CT 06840 (US)
(74) Representative: Simon, Elke Anna Maria
(86) International application number: PCT/EP2004/007634
(87) International publication number: WO 2005/014043

(56) References cited:
- EP-A1- 0 747 050
- EP-A1- 1 275 391
- WO-A-03/035029
- WO-A-03/059327
- WO-A-03/097045
- LACOURCIERE Y ET AL: "COMPARISON OF THE ANTIHYPERTENSIVE EFFECTS OF A FIXED DOSE COMBINATION OF TELMISARTAN AND HYDROCHLOROTHIAZIDE VERSUS TELMISARTAN MONOTHERAPY IN MILD TO MODERATE HYPERTENSIVE PATIENTS" CANADIAN JOURNAL OF CARDIOLOGY, PULSUS GROUP, INC, XX, vol. 16, no. SUPPL F, September 2000 (2000-09), page 107F, XP001053460 ISSN: 0828-282X
- CHRYSANTHAKOPOULOS S G: "Hypertension update: Low dose drug combination for the treatment of hypertension" HELLENIC JOURNAL OF CARDIOLOGY 1997 GREECE, vol. 38, no. 2, 1997, pages 73-83, XP008040900 ISSN: 1011-7970
- USHERWOOD T ET AL: "Early renal impairment: The role of the GP" MEDICINE TODAY 01 OCT 2002 AUSTRALIA, vol. 3, no. 10, 1 October 2002 (2002-10-01), pages 20-30, XP008040899 ISSN: 1443-430X
- MADI J C ET AL: "Valsartan alone and as part of combination therapy in general practice in Brazil.", INTERNATIONAL JOURNAL OF CLINICAL PRACTICE OCT 2001, vol. 55, no. 8, October 2001 (2001-10), pages 520-523, ISSN: 1368-5031

## Description

Thiazide diuretics cause the kidneys to increase the amount of salt and water eliminated from the body in the urine. They are classified by their chemistry and their specific pharmacological effect on the kidney. Originally used to treat patients with edema they were found to reduce blood pressure and became the first-line drug in antihypertensive therapy.

Chlorthalidone is usually grouped with the thiazides because it has the same pharmacological effect on the kidney even though it is chemically different. It's CAS name is 2-chloro-5-(2,3-dihydro-1-hydroxy-3-oxo-1h-isoindol-1-yl)benzene-sulfonamide having the following structure:

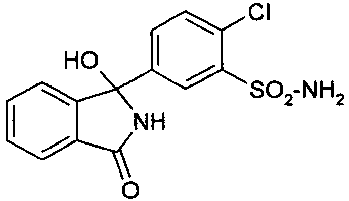

The precise mechanism by which thiazides reduce blood pressure is unknown. The addition of thiazide diuretics to ARB therapy has been shown to improve the ability of ARBs to lower BP.

While angiotensin II converting enzyme (ACE) inhibitors interfere with the generation of angiotensin II, ARBs lower blood pressure (BP) by specifically blocking angiotensin II activity at angiotensin II subtype 1 receptor sites independent of the pathway of angiotensin II generation. Adverse effects of ACE inhibitors which appear unrelated to angiotensin II blockade like cough and angioedema can be avoided with ARBs. They have been shown to provide cardiovascular and renal protection, independent of their effects on systemic BP. As is the case with other antihypertensive drugs, monotherapy with ARBs does not always reduce BP to recommended levels in hypertensive individuals, and ARBs show a limited dose-responsiveness, i.e. their BP-lowering effect often cannot be significantly improved by simply increasing the dose.

Lacourcière et al, Can J Cardiol 16:107F (2000), disclose that treatment with a fixed dose combination of 80mg telmisartan and 12.5mg hydrochlorothiazide confers additional blood pressure reduction, and WO 03/059327 published July 24, 2003 teaches hydrochlorothiazide as the preferred diuretic in fixed dose combinations with telmisartan.

WO 02/41890 (EP-A-1336407) discloses a pharmaceutical composition of olmesartan and a diuretic, particularly hydrochlorothiazide.

WO 03/035029 describes an oral dosage form which might also be used for drug combination products of an ACE inhibitor or angiotensin II antagonist with a diuretic. WO 03/097045 describes triple combinations of an angiotensin receptor blocker, a calcium channel blocker and a diuretic.

Finally, EP-A-0 747050 discloses oral formulations of irbesartan and optionally a divretic, which can be chlorthalidone, but is preferably HCTZ.

When combining ARBs with thiazide diuretics in hypertension therapy available thiazides such as bendrofluazide, bendroflumethiazide, benzthiazide, chlorothiazide, cyclopenthiazide, hydrochlorothiazide (HCTZ), hydroflumethiazide, indapamide, methyclothiazide, metolazone, polythiazide, quinethazone, trichlormethiazide or xipamide are generally considered equally effective. It is the merit of the present invention to identify chlorthalidone as a preferred thiazide diuretic for combination therapy if reduction of systolic BP is a major concern. Thus, by combining the sodium salt of the ARB telmisartan with chlorthalidone instead of hydrochlorothiazide, at least an additional 2-4 mmHg up to 10 mmHg reduction in systolic BP can be achieved. This appears particularly favourable when treating elderly people, since the prevalence of systolic hypertension increases with age. Additionally, the longer duration of the antihypertensive effect of chlorthalidone (mean plasma half-life of 40-60 hours) compared to hydrochlorothiazide (mean plasma half-life of about 24 hours) can be favourably combined with the action profile of the various ARBs to achieve an efficient 24-hour blood pressure control, which allows once-daily dosing without compromising BP control at the end of the dosing period.

Thus, it is an objective of the present invention to provide a pharmaceutical preparation or fixed dose combination comprising as active ingredient chlorthalidone and the sodium salt of telmisartan. Usually the combination consists of the two active ingredients in admixture with one or more excipients (adjuvants). Preparations are for oral administration such as tablets, sugar-coated tablets, capsules, powders, suspensions or suppositories. Preferred embodiments of tablets may comprise two or more layers.

Examples of suitable excipients (adjuvants) are mannitol, sorbitol, xylit, saccharose, calciumcarbonat, calciumphosphat, lactose, croscarmellose sodium salt (cellulose carboxymethylether sodium salt, cross linked), crospovidone, sodium starch glycolate, Hydroxypropylcellulose (low substituted), maize starch, polyvinylpyrrolidon, copolymers of vinylpyrrolidon with other vinylderivatives (copovidones), hydroxypropyl cellulose, hydroxypropylmethyl cellulose, microcristalline cellulose or starch, magnesiumstearat, sodiumstearylfumarat, talc, hydroxypropylmethylcellulose, carboxymethylcellulose, celluloseacetatphthalat, polyvinylacetat, water, water/ethanol, water/glycerine, water/sorbit, water/polyethylenglykol, propylenglykol, cetylstearylalkohol, carboxymethylcellulose or substances containing fat such as hard grease.

The various excipients are usually included for different purposes. Thus, the list of excipients comprises
- Inert diluents such as mannitol, sorbitol, xylit, saccharose, calciumcarbonat, calciumphosphat and lactose
- Disintegrants such as croscarmellose sodium salt (cellulose carboxymethylether sodium salt, cross linked), crospovidone, sodium starch glycolate, hydroxypropylcellulose (low substituted) and maize starch
- Binders such as polyvinylpyrrolidon, copolymers of vinylpyrrolidon with other vinylderivatives (copovidones), hydroxypropyl cellulose, hydroxypropylmethyl cellulose, microcristalline cellulose or starch
- Lubricants such as magnesiumstearat, sodiumstearylfumarat and talc
- Agents for delaying release such as hydroxypropylmethyl cellulose, carboxymethyl cellulose, celluloseacetatphthalat and polyvinyl acetate and
- Dyes acceptable for pharmaceutically use such as ferric oxides telmisartan

The amount of in a single dosage form is usually in the range of 10-800 mg. preferred ranges are 60-90 mg 30-60 mg 80-85 mg, 40-45 mg or 20-25 mg. The amount of chlorthalidone in a single dosage form is usually in the range of 10-15 mg or 20-30 mg, preferably 12-13 mg or 24-26 mg.

To produce a pharmaceutical preparation according to the invention such as a tablet for oral administration procedures known in the art can be used. Suitable excipients for the compression of ARBs with chlorthalidone after mixing are sorbitol and magnesiumstearat, which can be replaced by other excipients such as mannitol or saccharose. Occasionally the properties of tablets can be modified by granulation of an active ingredient with selected excipients before final compression of all the components of the pharmaceutical preparation. For this purpose the ARB or salt thereof can be mixed, for example, with mannitol, hydroxypropyl cellulose and, optionally, a coloring agent in a suitable blender. The resulting blend is preferably sieved and can be subjected to a dry granulation process in a roller compactor. The mentioned excipients might be replaced by other adjuvants such as lactose or microcristalline cellulose. The resulting granulate is mixed with chlorthalidone and other excipients such as mannitol, microcristalline cellulose, sodium starch glycolate, magnesium separate and optionally a coloring agent before being compressed to tablets. Alternatively, adjuvants such as lactose or croscarmellose sodium salt can be used.

It is often preferred that a fixed dose combination dosage form comprising an ARB and chlorthalidone shows fast dissolution and immediate drug release combined with adequate stability. In case mere combination of the active ingredients is not practical due to incompatibilities with excipients used in the mono-dosage form of the active ingredients, it is possible to coat chlorthalidone particles in a fluidized-bed granulator with a polymer solution containing water soluble polymers like hydroxypropyl-cellulose, hydroxypropylmethylcellulose or polyvinylpyrrolidone, thereby reducing the contact surface area of the chlorthalidone particles with the other components of the dosage form.

If this approach does not allow to stabilize the dosage form to a degree sufficient to achieve an acceptable shelf life or, due to the gel-forming properties of the polymer coat, slows the dissolution of chlorthalidone to an unacceptable rate, it is possible to produce separate film-coated tablets for the ARB and chlorthalidone in such a size and shape that these can be filled into a capsule. By dividing the doses into two to four single small tablets for the ARB and into one or two small tablets for chlorthalidone, a capsule of size 1 to 0 long can be filled.

If this approach, due to a lag-time effect of the large capsule shells, reduces the drug dissolution rate of the ARB too much or, with regard to patients' compliance, a zero long capsule is considered unreliable, it is possible to manufacture bilayer pharmaceutical tablets comprising a first layer containing the ARB in a pharmaceutical tablet base formulation having immediate release (fast dissolution) characteristics that readily dissolves in a physiological aqueous medium (dissolving tablet matrix), and a second layer containing chlorthalidone in a pharmaceutical tablet base formulation having immediate release characteristics that readily swells and disintegrates in a physiological aqueous medium (disintegrating tablet matrix). The composition of a bilayer tablet can be chosen such that it facilitates dissolution of the drug at a physiological pH level and provides for immediate release of chlorthalidone from the fast disintegrating matrix. At the same time, a bilayer tablet structure can reduce stability problems caused by an incompatibility of chlorthalidone with constituents of an ARB formulation.

The dissolving tablet matrix of the first tablet layer may have acidic, neutral or basic properties, a basic tablet matrix being preferred. In such preferred embodiments, the dissolving matrix comprises a basic agent, a water-soluble diluent and, optionally, other excipients (adjuvants). Specific examples of suitable basic agents are alkali metal hydroxides such as NaOH and KOH; basic amino acids such as arginine and lysine; and meglumine (N-methyl-D-glucamine), NaOH and meglumine being preferred.

Specific examples of suitable water-soluble diluents are carbohydrates such as monosaccharides like glucose; oligosaccharides like sucrose, anhydrous lactose and lactose monohydrate; and sugar alcohols like sorbitol, mannitol, dulcitol, ribitol and xylitol. Sorbitol is a preferred diluent.

Other excipients (adjuvants) are, for instance, selected from binders, carriers, fillers, lubricants, flow control agents, crystallization retarders, solubilizers, coloring agents, pH control agents, surfactants and emulsifiers, specific examples of which are given below in connection with the second tablet layer composition. The excipients and/or adjuvants for the first tablet layer composition are preferably chosen such that a non-acidic, fast dissolving tablet matrix is obtained.

Other (optional) constituents may, for instance, be chosen from one or more of the following excipients and/or adjuvants in the amounts indicated:
10 to 30 wt.%, preferably 15 to 25 wt.%, of binders, carriers and fillers, thereby replacing the water-soluble diluent;
0.1 to 5 wt. %, preferably 0.5 to 3 wt. %, of lubricants;
0.1 to 5 wt.%, preferably 0.3 to 2 wt.%, of flow control agents;
1 to 10 wt. %, preferably 2 to 8 wt. %, of crystallization retarders;
1 to 10 wt.%, preferably 2 to 8 wt.%, of solubilizers;
0.05 to 1.5 wt.%, preferably 0.1 to 0.8 wt.%, of coloring agents;
0.5 to 10 wt.%, preferably 2 to 8 wt.%, of pH control agents;
0.01 to 5 wt.%, preferably 0.05 to 1 wt.%, of surfactants and emulsifiers.

The fast disintegrating tablet matrix of the second tablet layer composition may comprise a filler, a binder, a disintegrant and, optionally, other excipients (adjuvants). The filler is preferably selected from anhydrous lactose, spray-dried lactose and lactose monohydrate.

The binder is selected from the group of dry binders and/or the group of wet granulation binders, depending on the manufacturing process chosen for the second tablet layer. Suitable dry binders are, for example, cellulose powder and microcrystalline cellulose. Specific examples of wet granulation binders are corn starch, polyvinyl pyrrolidone (Povidon), vinylpynolidone-vinylacetate copolymer (Copovidone) and cellulose derivatives like hydroxymethylcellulose, hydroxyethylcellulose, hydroxypropyl-cellulose and hydroxypropylmethylcellulose.

Suitable disintegrants are, e.g., sodium starch glycolate, Crospovidon, Croscarmellose, sodium carboxymethylcellulose and dried corn starch, sodium starch glycolate being preferred.

The other excipients and adjuvants, if used, are preferably selected from diluents and carriers such as cellulose powder, microcrystalline cellulose, cellulose derivatives like hydroxymethylcellulose, hydroxyethylcellulose, hydroxypropylcellulose and hydroxypropylmethylcellulose, dibasic calcium phosphate, corn starch, pregelatinized starch, polyvinyl pyrrolidone (Povidone) etc.; lubricants such as stearic acid, magnesium stearate, sodium stearylfumarate, glycerol tribehenate, etc.; flow control agents such as colloidal silica, talc, etc.; crystallization retarders such as Povidone, etc.; solubilizers such as Pluronic, Povidone, etc.; coloring agents, including dyes and pigments such as Iron Oxide Red or Yellow, titanium dioxide, talc, etc.; pH control agents such as citric acid, tartaric acid, fumaric acid, sodium citrate, dibasic calcium phosphate, dibasic sodium phosphate, etc.; surfactants and emulsifiers such as Pluronic, polyethylene glycols, sodium carboxymethyl cellulose, polyethoxylated and hydrogenated castor oil, etc.; and mixtures of two or more of these excipients and/or adjuvants.

Bilayer tablets with telmisartan can be produced with a method comprising the steps of:
(i) providing a first tablet layer composition by
   a) preparing an aqueous solution of the ARB, at least one basic agent and, optionally, a solubilizer and/or a crystallization retarder;
   b) spray-drying said aqueous solution to obtain a spray-dried granulate;
   c) mixing said spray-dried granulate with a water-soluble diluent to obtain a premix;
   d) mixing said premix with a lubricant to obtain a final blend for the first tablet layer;
   e) optionally, adding other excipients (adjuvants) in any of steps a) to d);
(ii) providing a second tablet layer composition by
   f) mixing and/or granulating chlorthalidone with the constituents of a disintegrating tablet matrix and, optionally, further excipients (adjuvants);
   g) admixing a lubricant to obtain a final blend for the second tablet layer;
(iii) introducing the first or the second tablet layer composition in a tablet press;
(iv) compressing said tablet layer composition to form a tablet layer;
(v) introducing the other tablet layer composition into the tablet press; and
(vi) compressing both tablet layer compositions to form a bilayer tablet.

Chlorthalidone is usually employed as a fine-crystalline powder, optionally in fine-milled, peg-milled or micronized form. For instance, the particle size distribution of chlorthalidone, as determined by the method of laser light scattering in a dry dispersion system (Sympatec Helos/Rodos, focal length 100 mm) is preferably as follows:
d₁₀ : ≤ 20 µm, preferably 2 to 10 µm
d₅₀ : 2 to 80 µm, preferably 10 to 30 µm
d₉₀ 5 to 150 µm, preferably 40 to 80 µm

The ARB is preferably dissolved and transformed into a substantially amorphous form, its initial crystal morphology and particle size being of little importance for the physical and biopharmaceutical properties of the bilayer tablet formulation obtained. It is however preferred to remove agglomerates from the starting material, e.g. by sieving, in order to facilitate wetting and dissolution during further processing.

A bilayer tablet generally comprises 10-800 mg, preferably 20-100 mg of telmisartan and 6.25-50 mg, preferably 12.5-25 mg of chlorthalidone. Particularly preferred forms are bilayer tablets comprising about 20/12.5 mg, 40/12.5 mg, 80/12.5 mg, 160/12.5 mg, 20/25 mg, 40/25 mg, 80/25 mg and 160/25 mg of and chlorthalidone, respectively.

The first tablet layer composition generally comprises 3 to 50 wt.%, preferably 5 to 35 wt.%, of active ingredient; 0.25 to 20 wt.%, preferably 0.40 to 15 wt.%, of basic agent; and 30 to 95 wt.%, preferably 60 to 80 wt.% of water-soluble diluent.

The second tablet layer composition generally comprises 1.5 to 35 wt.%, preferably 2 to 15 wt.%, of active ingredient; 25 to 75 wt.%, preferably 35 to 65 wt.%, of filler; 10 to 40 wt.%, preferably 15 to 35 wt.%, of dry binder; 0.5 to 5 wt.%, preferably 1 to 4 wt.%, of wet granulation binder; and 1 to 10 wt.%, preferably 2 to 8 wt.%, of disintegrant. The other excipients and adjuvants are generally employed in the same amount as in the first tablet layer composition.

For preparing the bilayer tablet according to the present invention, the first and second tablet layer compositions may be compressed in the usual manner in a bilayer tablet press, for example a high-speed rotary press in a bilayer tableting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN. During bilayer tablet compression adequate bond formation between the two layers is achieved by virtue of distance attraction forces (intermolecular forces) and mechanical interlocking between the particles.

Bilayer tablets obtained release the active ingredients rapidly and in a largely pH-independent fashion, with complete release occurring within less than 90 min and release of the major fraction occurring within less than 30 min. The dissolution/ disintegration kinetics of the bilayer tablet may be controlled in different ways. For instance, both layers may dissolve/disintegrate simultaneously. Preferably, however, the second tablet layer containing chlorthalidone disintegrates first whereas the first tablet layer containing the ARB dissolves in parallel or subsequently.

With the following method of producing a bilayer tablet, particularly favourable dissolution/disintegration and drug release properties are achieved Said method comprises
(i) providing a first tablet layer composition by
   a) preparing an aqueous solution of telmisartan , at least one basic agent and, optionally, a solubilizer and/or a crystallization retarder;
   b) spray-drying said aqueous solution to obtain a spray-dried granulate;
   c) mixing said spray-dried granulate with a water-soluble diluent to obtain a premix;
   d) mixing said premix with a lubricant to obtain a final blend for the first layer;
   e) optionally, adding other excipients and/or adjuvants in any of steps a) to d);
(ii) providing a second tablet layer composition by
   f) mixing and /or granulating chlorthalidone with the constituents of a disintegrating tablet matrix and, optionally, further excipients and/or adjuvants;
   g) admixing a lubricant to obtain a final blend for the second tablet layer;
(iii) introducing the first or the second tablet layer composition into a tablet press;
(iv) compressing said tablet layer composition to form a tablet layer;
(v) introducing the other tablet layer composition into the tablet press; and
(vi) compressing both tablet layer compositions to form a bilayer tablet.

In a preferred embodiment of this method, an aqueous alkaline solution of ARB is prepared by dissolving the active ingredient in purified water with the help of one or more basic agents like sodium hydroxide and meglumine. Optionally, a solubilizer and/or a recrystallization retarder may be added. The dry matter content of the starting aqueous solution is generally 10 to 40 wt.%, preferably 20 to 30 wt.%.

The aqueous solution is then spray-dried at room temperature or preferably at increased temperatures of, for instance, between 50 and 100°C in a co-current or counter current spray-drier at a spray pressure of, for instance, 1 to 4 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value of between about 80°C and 90°C while the other process parameters such as spray pressure, spraying rate, inlet air temperature, etc. are adjusted accordingly.

The spray-dried granulate obtained is preferably a fine powder having the following particle size distribution:
d₁₀ : ≤ 20 µm, preferably ≤10 µm
d₅₀ : ≤ 80 µm, preferably 20 to 55 µm
d₉₀ : ≤ 350 µm, preferably 50 to 150 µm

After spray-drying the ARB as well as the excipients contained in the spray-dried granulate are in a substantially amorphous state with no crystallinity being detectable. From a physical point of view, the spray-dried granulate containing telmisartan is a solidified solution or glass having a glass transition temperature Tg of preferably > 50°C, more preferably > 80°C. Based on 100 parts by weight of ARB, the spray-dried granulate preferably contains 5 to 200 parts by weight of basic agent and, optionally, solubilizer and/or crystallization retarder.

The water-soluble diluent is generally employed in an amount of 30-95 wt.%, preferably 60-80 wt.%, based on the weight of the first tablet layer composition.

The lubricant is generally added to the premix in an amount of 0.1 to 5 wt.%, preferably 0.3 to 2 wt.%, based on the weight of the first tablet layer composition.

Mixing is carried out in two stages, i.e. in a first mixing step the spray-dried granulate and the diluent are admixed using , for example, a high-shear mixer or a free-fall blender, and in a second mixing step the lubricant is blended with the premix, preferably also under conditions of high shear. The method of the invention is however not limited to these mixing procedures and, generally, alternative mixing procedures may be employed in steps c), d), and also in the subsequent steps f) and g), such as, for example, container mixing with intermediate screening.

For direct compression, the second tablet layer composition may be prepared by dry-mixing the constituent components, for example by means of a high-intensity mixer or a free-fall blender. Alternatively and preferably, the second tablet layer composition is prepared using a wet granulation technique wherein an aqueous solution of a wet granulation binder is added to a premix and subsequently the wet granulate obtained is dried, e.g. in a fluidized-bed dryer or drying chamber. The dried mixture is screened and then a lubricant is admixed, for example using a tumbling mixer or free-fall blender, where after the composition is ready for compression.

For production of the bilayer tablet according to the present invention, the first and second tablet layer compositions are compressed in a bilayer tablet press, e.g. a rotary press in the bilayer tableting mode, in the manner described above.

According to the present invention the sodium salt of telmisartan and chlorthalidone are combined to produce pharmaceutical preparations for the treatment of hypertension, and treatment or prevention of cardiovascular events or stroke. Preferably, use of said combinations is indicated in connection with hypertension, cardiac insufficiency (heart failure), ischemic peripheral circulatory disorders, myocardial ischemia, myocardial infarction, left ventricular hypertrophy, diabetic neuropathy, diabetic retinopathy, glaucoma , diabetic nephropathy, diseases of the bladder or gastrointestinal diseases. The combinations appear particularly valuable to prevent, i.e. to reduce the risk of transient ischemic attacks, stroke, myocardial infarction, left ventricular hypertrophy, progression of cardiac insufficiency after cardiovascular events such as myocardial infarction, development of diabetic nephropathy, diabetic retinopathy, diabetic neuropathy and any kind of cardiovascular death.

The following examples 1 and 2 and 4-6 describe selected embodiments of the present invention.

### EXAMPLES

### Example 1

In a first step the sodium salt of the ARB telmisartan, mannitol, red iron oxide and hydroxypropyl cellulose are mixed with a high-shear mixer. Subsequently magnesium stearate, which has been passed through an 0. 8 mm sieve, is added and the mixture subjected to a dry granulation process in a roller compactor.

In a parallel process step chlorthalidone is mixed with mannitol, microcristalline cellulose, sodium starch glycolate and red iron oxide. Both the chlorthalidone mixture and the granules containing the sodium salt of telmisartan are passed through an 0.8 mm sieve, intermixed in a free fall blender and finally mingled with magnesium stearate, which had been passed through an 0.8 mm sieve.

One obtains a pharmaceutical preparation which can be conveniently compressed to tablets showing a good solubility of the active ingredients. Using a suitable tablet press (e.g. Korsch EKO or Fette P1200) produces tablets with the following composition, whereas the amount of sodium salt of telmisartan corresponds to an amount of 80 mg of the free acid of telmisartan.

| **Constituents** | **mg/tablet** | **%tablet** |
|---|---|---|
| Sodium salt of telmisartan | 83.417 | 13.903 |
| Chlorthalidone | 12.500 | 2.083 |
| Mannitol | 336.483 | 56.081 |
| Microcrystalline cellulose | 120.000 | 20.000 |
| Sodium starch glycolate | 30.000 | 5.000 |
| Red iron oxide | 0.600 | 0.100 |
| Hydroxypropylcellulo se | 5.000 | 0.833 |
| Magnesiumstearate | 12.000 | 2.000 |
| **Total** | **600.000** | **100.000** |

Similarly, the composition of the tablet can be described as follows:

| **Constituents** | **mg/tablet** | **%/tablet** | **%/granules** |
|---|---|---|---|
| Sodium salt of telmisartan | 83.417 | 13.903 | 83.417 |
| Mannitol | 10.983 | 1.831 | 10.983 |
| Hydroxypropylcellulo se | 5.000 | 0.833 | 5.000 |
| Red iron oxide | 0.100 | 0.017 | 0.100 |
| Magnesiumstearate | 0.500 | 0.083 | 0.500 |
| **Total** | **100.000** | **16.667** | **100.000** |
| Chlorthalidone | 12.500 | 2.083 | |
| Mannitol | 325.500 | 54.250 | |
| Microcrystalline cellulose | 120.000 | 20.000 | |
| Sodium starch glycolate | 30.000 | 5.000 | |
| Red iron oxide | 0.500 | 0.083 | |
| Magnesiumstearate | 11.500 | 1.917 | |
| **Total** | **600.000** | **100.000** | |

The tablets show the following features:
Size: 16.2 x 7.9 mm (r=5.86 mm)
Weight: 598.7 mg ± 0.22 %
Thickness: on the average 6.16 mm

### Example 2

Chlorthalidone, the sodium salt of telmisartan, sorbitol and red iron oxide are mixed in a free fall blender, passed through a 0.8 mm sieve, and processed to a powder after the addition of magnesium stearate. Using a suitable tablet press (e.g. Korsch EKO or Fette P1200) tablets of, the following composition are obtained, whereas the amount of sodium salt of telmisartan corresponds to an amount of 80 mg of the free acid of telmisartan.

| **Constituents** | **mg/tablet** | **%tablet** |
|---|---|---|
| Sodium salt of telmisartan | 83.417 | 13.903 |
| Chlorthalidone | 12.500 | 2.083 |
| Sorbitol | 494.483 | 82.414 |
| Red iron oxide | 0.600 | 0.100 |
| Magnesiumstearate | 9.000 | 1.500 |
| **Total** | **600.000** | **100.000** |

Typical features of the tablets are:
Size: 16.2 x 7.9 mm (r=5.86 mm)
Weight: 600.7 mg ± 0.34 %
Thickness: durchschnittlich 5.96 mm

### 1. Spray solution

225.000 kg of purified water are measured into a suitable stainless steel vessel at a temperature of between 20-40°C. In sequence, 3.780 of kg sodium hydroxide, 45.000 kg of telmisartan (mixture of polymorph A and B), 13.500 kg of Povidone K 25 and 13.500 kg of meglumine are dissolved in the purified water under intensive stirring until a virtually clear, slightly yellowish, alkaline solution is obtained.

### 2. Spray drying

The solution is sprayed into a suitable spray dryer, e.g. a Niro P 6.3 equipped with Schlick atomizing nozzles of 1.0 mm diameter, with a flow-through heating coil connected upstream of the dryer, and dried to give a white to off-white fine granulate. The spray mode is countercurrent at a spray-pressure of about 3 bar, an inlet air temperature of about 125°C and a spray rate of about 11 kg/h, thus resulting in an outlet air temperature of about 85°C. The temperature of the flow through heating coil water bath is set at a temperature of about 80°C.

### 3. - Protective Screening

The dry granulate powder is screened through a screen of 0.5 mm mesh size, e.g. using a Vibra Sieve machine.

The resulting amorphous telmisartan spray-dried granulate is further processed to the first layer of a bilayer tablet composition similar to example 4.

### Example 4

| | **Constituents** | **mg/tablet 1st layer** | **mg/SD granulate** | **mg/tablet 2nd layer** |
|---|---|---|---|---|
| (01) | Telmisartan SD granulate consisting of (02) to (06): | 67.360 | | |
| (02) | Telmisartan | | 40.000 | |
| (03) | Sodium hydroxide | | 3.360 | |
| (04) | Polyvidone | | 12.000 | |
| (05) | Meglumine | | 12.000 | |
| (06) | Purified water | | 264.000* | |
| (07) | Sorbitol P/6 | 168.640 | | |
| (08) | Magnesium stearate, screened | 4.000 | | 1.000 |
| (09) | Chlorthalidone | | | 12.500 |
| (10) | Microcrystalline cellulose | | | 64.000 |
| (11) | Red iron oxide | | | 0.330 |
| (12) | Sodium starch glycolate | | | 4.000 |
| (13) | Lactose monohydrate fine, screened | | | 112.170 |
| (14) | Maize starch, dried at 45°C | | | 6.000 |
| | | 240.000 | 67.360 | 200.000 |

| | | | | |
|---|---|---|---|---|
| * 200 mg in SD granulate, 64 mg in granulation liquid of chlorthalidone granulate | | | | |

### 1. Final blend A

168.640 kg of sorbitol are mixed with 67.360 kg of telmisartan spray dried granulate in a suitable high shear mixer, e.g. Diosna P 600, for 4 minutes using both impeller and chopper. Next 4.0 kg of magnesium stearate are added to the resulting pre-mix and admixed in the high shear mixer for further 30 seconds.

### 2. Final blend B

9.000 kg of purified water of about 70°C are transferred to a suitable mixing vessel, 6.000 kg of maize starch, dried at 45°C, are suspended in the water. This suspension is stirred into 55.000 kg of purified water of about 90°C using e.g. an Ekato stirrer.

Next, 112.170 kg of lactose monohydrate, 12.500 kg of chlorthalidone, 64.000 kg of microcrystalline cellulose (Avicel PH 101), 0.330 kg of red iron oxide and 4.000 kg of sodium starch glycolate are mixed in a suitable high shear granulator, e.g. Diosna P 600, until homogeneous, and moistened with 70.000 kg of the above-prepared aqueous granulating liquid.

Process parameters for wet granulation:

| **Process step** | **Duration (min)** | **Impeller (setting)** | **Chopper (setting)** |
|---|---|---|---|
| Pre-mixing | 3 | 1 | 1 |
| Moistening | 2 | 1 | 1 |
| Wet mixing | 4 | 2 | 2 |
| Emptying | About 0.5 | 1 | 0 |

After moistening, the resulting wet granulate is dried in a suitable fluid bed dryer, e.g. Glatt WSG 120 at an inlet air temperature of 100°C, an inlet air flow of 2000-3000 m³/h until a product temperature of about 55°C is reached.

The dry granulate is screened to reduce the particle size using a suitable screening machine, e.g. a Comil screen machine equipped with a rasp screen of 2 mm mesh size.

Finally 1.000 kg of pre-screened magnesium stearate are admixed to the screened granulate material and mixed in a suitable tumbling mixer, e.g. a Lermer rotating spike mixer, for 100 revolutions at a speed of 8-10 rpm.

### 3. Bilayer tablet compression

Using a suitable rotary tablet press, 240 kg of the final blend (A) and 200 kg of the final blend (B) are compressed into bilayer tablets. The target weight for the first layer is 240 mg, the target weight for the second layer is 200 mg.

Process parameters for tableting:

| | | |
|---|---|---|
| Tablet press | Fette 3090 | |
| Tabletting speed | 100.000 (80.000 - 120.000) tablets/h | |
| Stirrer blade speed: | 1st layer about 30 rpm | 2nd layer about 75 rpm |
| Compression force | 5 (4-6) KN | 12 (10-14) KN |

As a rule, the tablet hardness is adjusted by variation of the main compression force of the second layer.

The resulting bilayer tablets have the following characteristics:

| | |
|---|---|
| Shape / diameter | oval, both faces convex / 14 x 6.8 mm |
| Colour | first layer: white to off-white |
| | second layer: red |
| Weight | 440 mg (total) |
| | 240 mg (layer 1: with telmisartan) |
| | 200 mg (layer 2: with chlorthalidone) |
| Thickness | about 5.2 mm |

### Example 5

| | **Constituents** | **mg/tablet 1st layer** | **mg/SD granulate** | **mg/tablet 2nd layer** |
|---|---|---|---|---|
| (01) | Telmisartan SD granulate consisting of (02) to (06): | 67.360 | | |
| (02) | Telmisartan | | 40.000 | |
| (03) | Sodium hydroxide | | 3.360 | |
| (04) | Polyvidone | | 12.000 | |
| (05) | Meglumine | | 12.000 | |
| (06) | Purified water | | (200.000) | |
| (07) | Sorbitol P/6 | 168.640 | | |
| (08) | Magnesium stearate, screened | 4.000 | | 1.000 |
| (09) | Chlorthalidone | | | 25.000 |
| (10) | Microcrystalline cellulose | | | 64.000 |
| (11) | Yellow iron oxide | | | 0.330 |
| (12) | Sodium starch glycolate | | | 4.000 |
| (13) | Lactose monohydrate fine | | | 105.67 |
| | | 240.000 | 67.360 | 200.000 |

Manufacturing is carried out as in Example 3. Instead of the wet granulation process described in Example 2, the second layer composition is manufactured by dry mixing of (09) to (13) in a suitable free fall blender, e.g. a 1 m³ container mixer, for 200 revolutions at a speed of 10 rpm. Then, (08) is admixed to the main mixture for further 50 revolutions in the container mixer. In order to achieve a homogenous distribution of the color pigment, an additional premix with yellow iron oxide and a portion of the microcrystalline cellulose, e.g. 2.000 kg, which is screened through an 0.8 mm mesh screen manually before transfer to the main mixture, may be performed. The resulting bilayer tablets display virtually the same physical characteristics as described in example 3, except for the color.

### Example 6

Composition of Telmisartan/Chlorthalidone Bilayer Tablets (mg per tablet):

| **Ingredient** | | **40/12.5 mg** | **80/12.5 mg** |
|---|---|---|---|
| **Telmisartan layer** | | | |
| | Telmisartan | 40.000 | 80.000 |
| | Sodium hydroxide | 3.360 | 6.720 |
| | Povidone | 12.000 | 24.000 |
| | Meglumine | 12.000 | 24.000 |
| | Purified water* | (200.000) | (400.000) |
| | Sorbitol | 168.640 | 337.280 |
| | Magnesium stearate | 4.000 | 8.000 |
| Total telmisartan layer | | 240.000 | 480.000 |

| **Chlorthalidone layer** | | | |
|---|---|---|---|
| | Chlorthalidone | 12.500 | 12.500 |
| | Lactose monohydrate | 112.170 | 112.170 |
| | Microcrystalline Cellulose | 64.000 | 64.000 |
| | Corn starch | 6.000 | 6.000 |
| | Red iron oxide | 0.330 | 0.330 |
| | Sodium starch glycolate | 4.000 | 4.000 |
| | Purified water* | (64.000) | (64.000) |
| | Magnesium stearate | 1.000 | 1.000 |
| Total Chlorthalidone layer | | 200.000 | 200.000 |
| **Total tablet weight** | | 440.000 | 680.000 |

| | | | |
|---|---|---|---|
| *Does not appear in final product | | | |

## Claims

1. A pharmaceutical preparation comprising as therapeutic ingredients chlorthalidone and the sodium salt of telmisartan, **characterised in that** it is to be administered orally.

2. The preparation of claim 1, consisting of the therapeutic ingredients in admixture with one or more excipients.

3. The preparation of claim 2, wherein the excipient is selected from the group consisting of mannitol, sorbitol, xylit, saccharose, calciumcarbonat, calciumphosphat, lactose,
croscarmellose sodium salt (cellulose carboxymethylether sodium salt, cross linked), crospovidone, sodium starch glycolate, Hydroxypropylcellulose (low substituted), maize starch, polyvinylpyrrolidon, copolymers of vinylpyrrolidon with other vinylderivatives (copovidones), hydroxypropyl cellulose, hydroxypropylmethyl cellulose, microcristalline cellulose or starch, magnesiumstearat, sodiumstearylfumarat, talc, hydroxypropylmethylcellulose, carboxymethylcellulose, celluloseacetatphthalat, polyvinylacetat, water, water/ethanol, water/glycerine, water/sorbit, water/polyethylenglykol, propylenglykol, cetylstearylalkohol, carboxymethylcellulose or substances containing fat such as hard grease.

4. Use of the active ingredients chlorthalidone and
the sodium salt of telmisartan for the preparation of a pharmaceutical composition to treat hypertension in an individual in need thereof.

5. The use of claim 4 to additionally treat or prevent cardiovascular events, stroke, cardiac insufficiency (heart failure), ischemic peripheral circulatory disorders, myocardial ischemia, myocardial infarction, left ventricular hypertrophy, diabetic neuropathy, diabetic retinopathy, glaucoma , diabetic nephropathy, diseases of the bladder or gastrointestinal diseases.

6. The use of claim 4 to additionally reduce the risk of transient ischemic attacks, stroke, myocardial infarction, progression of cardiac insufficiency after cardiovascular events such as myocardial infarction, left ventricular hypertrophy, development of diabetic nephropathy, diabetic retinopathy, diabetic neuropathy and any kind of cardiovascular death.

## Patentansprüche

1. Pharmazeutische Zubereitung, umfassend als therapeutische Bestandteile Chlorthalidon und das Natriumsalz von Telmisartan, **dadurch gekennzeichnet, dass** es oral verabreicht wird.

2. Zubereitung nach Anspruch 1, bestehend aus den therapeutischen Bestandteilen in Mischung mit ein oder mehr Hilfsstoffen.

3. Zubereitung nach Anspruch 2, wobei die Hilfstoffe ausgewählt sind aus der Gruppe, bestehend aus Mannitol, Sorbitol, Xylit, Saccharose, Calciumcarbonat, Calciumphosphat, Lactose, Croscarmellosenatriumsalz (Cellulosecarboxymethylethernatriumsalz, vernetzt), Crospovidon, Natriumstärkeglycolat, Hydroxypropylcellulose (gering substituiert), Maisstärke, Polyvinylpyrrolidon, Copolymere von Vinylpyrrodlidon mit anderen Vinylderivaten (Copovidone), Hydroxypropylcellulose, Hydroxypropylmethylcellulose, mikrokristalline Cellulose oder Stärke, Magnesiumstearat, Natriumstearylfumarat, Talk, Hydroxypropylmethylcellulose, Carboxymethylcellulose, Celluloseacetatphthalat, Polyvinylacetat, Wasser, Wasser/Ethanol, Wasser/Glycerin, Wasser/Sorbit, Wasser/Polyethylenglykol, Propylenglycol, Cetylstearylalkohol, Carboxymethylcellulose oder Fett enthaltende Substanzen, wie Hartfett.

4. Verwendung der aktiven Bestandteile Chlorthalidon und das Natriumsalz von Telmisartan zur Herstellung einer pharmazeutischen Zusammensetzung zur Behandlung von Bluthochdruck bei einem Individuum mit Bedarf hierfür.

5. Verwendung nach Anspruch 4 zur zusätzlichen Behandlung oder Vorbeugung von kardiovaskulären Vorfälle, Schlaganfall, kardialer Insuffizienz (Herzversagen), ischämischen Störungen des peripheren Kreislaufs, Myokardischämie, Myokardinfarkt, linksventrikulärer Hypertrophie, diabetischer Neuropathie, diabetischer Retinopathie, Glaukom, diabetischer Nephropathie, Erkrankungen der Blase oder Gastrointestinalerkrankungen.

6. Verwendung nach Anspruch 4 zur zusätzlichen Verringerung des Riskos von transienten ischämischen Anfällen, Schlaganfall, Myokardinfarkt, Fortschreiten von kardialer Insuffizienz nach kradiovaskulären Vorfällen, wie Myokardinfarkt, linksventrikulärer Hypertrophie, der Entwicklung von diabetischer Nephropathie, diabetischer Retinopathie, diabetischer Neuropathie und jeglicher Art von kardiovaskulärem Tod.

## Revendications

1. Préparation pharmaceutique comprenant en tant qu'ingrédients thérapeutiques de la chlorthalidone et le sel de sodium de telmisartan, **caractérisée en ce qu'**elle doit être administrée par voie orale.

2. Préparation selon la revendication 1, constituée des ingrédients thérapeutiques en mélange avec un ou plusieurs excipients.

3. Préparation selon la revendication 2, dans laquelle l'excipient est choisi dans le groupe constitué du mannitol, du sorbitol, du xylitol, du saccharose, du carbonate de calcium, du phosphate de calcium, du lactose, du sel de croscarmellose sodique (sel de carboxyméthyléther de cellulose sodique, réticulé), de la crospovidone, du glycolate d'amidon de sodium, de l'hydroxypropylcellulose (à faible degré de substitution), de l'amidon de maïs, de la polyvinyipyrrolidone, des copolymères de vinylpyrrolidone et d'autres dérivés vinyliques (copovidones), de l'hydroxypropylcellulose, de l'hydroxypropylméthylcellulose, de la cellulose ou de l'amidon microcristallins, du stéarate de magnésium, du stéarylfumarate de sodium, du talc, de l'hydroxypropylméthylcellulose, de la carboxyméthylcellulose, de l'acétophtalate de cellulose, de l'acétate de polyvinyle, de l'eau, de l'eau/éthanol, de l'eau/glycérine, de l'eau/sorbitol, de l'eau/polyéthylène glycol, du propylène glycol, de l'alcool cétylstéarylique, de la carboxyméthylcellulose ou des substances contenant des graisses telles que la graisse dure.

4. Utilisation des ingrédients actifs de chlorthalidone et de sel de sodium de telmisartan pour la préparation d'une composition pharmaceutique destinée à traiter l'hypertension chez un individu qui le nécessite.

5. Utilisation selon la revendication 4 pour en outre traiter ou prévenir les événements cardiovasculaires, l'AVC, l'insuffisance cardiaque, les troubles circulatoires périphériques ischémiques, l'ischémie myocardique, l'infarctus du myocarde, l'hypertrophie ventriculaire gauche, la neuropathie diabétique, la rétinopathie diabétique, le glaucome, la néphropathie diabétique, les maladies de la vessie ou les maladies gastro-intestinales.

6. Utilisation selon la revendication 4 pour en outre réduire le risque d'accidents ischémiques transitoires, d'AVC, d'infarctus du myocarde, de progression de l'insuffisance cardiaque suite à des événements cardiovasculaires tels que l'infarctus du myocarde, l'hypertrophie ventriculaire gauche, le développement de la néphropathie diabétique, de la rétinopathie diabétique, de la neuropathie diabétique et de tout type de décès d'origine cardiovasculaire.
